# EUROPÄISCHE PATENTANMELDUNG

(11) **EP 1 967 173 A1**
(43) Veröffentlichungstag der Anmeldung: **10.09.2008**
(21) Anmeldenummer: 07103635.4
(22) Anmeldetag: 06.03.2007
(51) Int. Cl.: A61K 8/04, A61K 8/92, A61Q 19/06, A61Q 19/10, C11D 1/00, C11D 7/00

(54) **Hautreinigungsmittel mit Partikel enthaltend hydriertes Rizinusöl**

(71) Anmelder: Peter Greven Hautschutz GmbH & Co. KG, 53902 Bad Münstereifel (DE)
(72) Erfinder: Stolz, Hermann-Josef, 53902 Bad Münstereifel (DE); Börnicke, Robert, 53902 Bad Münstereifel (DE); Matzel, Manfred, 50935 Köln (DE)
(74) Vertreter: Schreiber, Christoph

(57) **Zusammenfassung**

Hautreinigungsmittel enthaltend
- 2 bis 25 Gew.-% Reinigungskörper mit einer mittleren Korngröße von 100 bis 1.000 µm enthaltend hydriertes Rizinusöl,
- 2 bis 30 Gew.-% Tenside,
- 0,1 bis 10 Gew.-% Verdickungsmittel,

Wasser, sowie gegebenenfalls weiteren Hilfsstoffe.

## Beschreibung

Die Erfindung betrifft ein Hautreinigungsmittel, einen Reinigungskörper für das Hautreinigungsmittel sowie Verfahren zur Herstellung des Reinigungskörpers.

Hautreinigungsmittel können zur Verbesserung der Reinigungsleistung Abrasiva enthalten, die die Reinigungswirkung von tensidartigen Komponenten mechanisch unterstützen.

Zahlreiche anorganische und organische Abrasiva sind bekannt, die beispielsweise in Handreinigern oder Peeling-Präparaten eingesetzt werden.

Die zuerst verwendeten Abrasiva waren auf Basis von mineralischen Komponenten wie Quarzsand oder Bimsmehl. Nachteilig hieran ist jedoch, dass Quarzsand und Bimsmehle harte, scharfkantige Körner haben, was zu einer schlechten Hautverträglichkeit führt. Aufgrund ihrer hohen Dichte neigen sie zur Sedimentation und führen damit zu einem Verstopfen der Abflüsse.

Weitere eingesetzte Komponenten sind Holzmehle. Diese haben eine verbesserte Hautverträglichkeit und sedimentieren in den Abflüssen nicht mehr. Allerdings besteht durch die Verwendung von Holzmehl grundsätzlich das Risiko von Allergien gegen Terpeninhaltsstoffe des Holzes. Darüber hinaus ist nachteilig, dass Holzmehl In Verdacht steht, krebserregend zu sein, so dass strenge Sicherheitsvorkehrungen bei der Herstellung und Verarbeitung von Holzmehl zu beachten sind.

Weitere Abrasiva sind auf das Basis von Polyurethan- oder Polyethylenmehl erhältlich. Von ihrer Hautverträglichkeit, der Härte, etc. sind sie hervorragend als Abrasiva geeignet. Allerdings sind sie praktisch biologisch nicht abbaubar, so dass sie in Bezug auf Umweltschutz und Nachhaltigkeit nicht optimal sind.

Eine weitere Gruppe von Abrasiva sind auf der Basis von Mehlen von Naturstoffen erhältlich.

Die DE 40 38 076 C2 beschreibt beispielsweise Schalen- und Kernmehle, insbesondere Walnussschalenmehle.

EP 1 106 173 A2 beschreibt den Einsatz von Maiskolbenmehlen als Abrasiva.

Hierbei ist zu bedenken, dass zum Einsatz eine Desinfektion des eingesetzten Mehles notwendig ist, um eine Verkeimung der Produkte zu vermeiden. Die oben genannte EP 1 106 173 beschreibt ein Bleichverfahren für Naturmehle, das gleichzeitig gegebenenfalls vorhandene Keime abtötet. Während grundsätzlich die Verwendung solcher Naturstoffmehle unter Nachhaltigkeitsgesichtspunkten gut geeignet ist, sind die Eigenschaften solcher Mehle weiterhin nicht optimal.

Der Grad der Verschmutzung an den verschiedenen Arbeitsplätzen in der Industrie nimmt durch den gestiegenen Anspruch an Arbeitsicherheit und -hygiene ab. Daher besteht die Forderung, nicht zuletzt von Berufsdermatologen, nach hautfreundlicheren Produkten. Im Fokus stehen dabei vor allem die Reibemittel.

Es besteht immer noch ein Bedarf an Hautreinigungsmitteln, die gute Reinigungsleistungen bieten, jedoch verbesserte, hautschonende Eigenschaften aufweisen.Aufgabe der vorliegenden Erfindung war es, einen Reinigungskörper und ein Hautreinigungsmittel enthaltend den Reinigungskörper bereit zu stellen, die die genannten Nachteile des Standes der Technik überwindet, insbesondere eine hohe Hautverträglichkeit mit sich bringen.

Gelöst wird die Aufgabe durch ein Hautreinigungsmittel enthaltend
- 2 bis 25 Gew.-% eines Reinigungskörpers mit einer mittleren Korngröße von 100 bis 1.000 µm enthaltend hydriertes Rizinusöl,
- 2 bis 30 Gew.-% Tenside,
- 0,1 bis 10 Gew.-% Verdickungsmittel,
- Wasser, sowie gegebenenfalls weiteren Hilfsstoffe.

Erfindungsgemäß werden Reinigungskörper eingesetzt, die Rizinusöl, d.h. das aus *Rizinus communis* Samen gewonnene Öl, das anschließend zumindest teilweise hydriert wird, enthalten.

Bevorzugt besteht der erfindungsgemäße Reinigungskörper aus mindestens 20% Rizinusöl, bevorzugt mindestens 50% Rizinusöl und am meisten bevorzugt mindestens 90 Gew.-% Rizinusöl.

Der Grad der Hydrierung kann durch die Bestimmung der Jodzahl ermittelt werden. Besonders geeignet haben sich Rizinusöle mit einer Jodzahl von 0 bis 20 mg I₂/100g erwiesen.

Überraschenderweise zeigen die erfindungsgemäßen Reinigungskörper ein schmutzlöse- und schmutzabsorbierendes Verhalten. Schmutzpartikel bilden beim Einsatz des erfindungsgemäßen Reinigungsmittels eine Art Umhüllung um die erfindungsgemäßen Reinigungskörper. Auf diesem Wege wird eine gute Reinigungswirkung erreicht.

Die erfindungsgemäßen Reinigungskörper zeigen keinen oder nur untergeordneten abrasiven (abschabenden) Effekt, das erfindungsgemäße Reinigungsmittel ist daher besonders hautschonend.

Grundsätzlich können die Reinigungskörper neben dem hydrierten Rizinusöl auch noch weitere Stoffe, insbesondere Wachse enthalten. Besonders geeignet sind Paraffinwachse, Carnaubawachse, Candelilawachse, Polyethylenwachse, oxidierte Polyethylenwachse oder Mischungen davon. Es ist darauf zu achten, solche Wachse zu wählen, die einen Schmelzbereich oberhalb von 40°C, bevorzugt oberhalb von 80°C aufweisen.

Überraschenderweise zeigt sich, dass die Reinigungskörper auch in Gegenwart von Lösungsmitteln wie dibasischem Ester (DBE) stabil sind. Damit können die Reinigungskörper auch in Spezialprodukten wie Lackreinigern eingesetzt werden.

Überraschenderweise zeigt sich, dass sich die eingesetzten Reinigungskörper trotz der Anwesenheit der Tenside im Hautreinigungsmittel nicht auflösen.

Überraschenderweise zeigt sich auch, dass der erfindungsgemäß eingesetzte Reinigungskörper unter den üblichen Transport- und Lagerungsbedingungen stabil bleiben.

Als Tenside zur Verwendung im Hautreinigungsmittel haben sich insbesondere nicht-ionische, zwitter-ionische und anionische Tenside als geeignet erwiesen, beispielsweise Ethersulfate, Betaine, Alkylsulfonate, Succinate, Alkypolygylcoside, Eiweißfettsäurekondensate, Polyglycolether, Seifen und Mischungen davon. Die Auswahl entsprechender Tenside ist dem Fachmann geläufig.

Als Verdickungsmittel eignen sich insbesondere Bentonite, Xanthane, Acrylate, Alginate, Celluloseether, Carrageen und Mischungen davon.

Üblicherweise kann das erfindungsgemäße Hautreinigungsmittel einen oder mehrere der folgenden üblichen Zusatzstoffe enthalten:
- Rückfetter,
- Farbstoffe,
- Parfümstoffe,
- Titandioxid,
- Puffersubstanzen,
- Konservierungsmittel,
- flüssige Parafine,
- Glycerin,
- Antioxidationsmitteln.

Durch den Gehalt an Verdickungsmitteln und den Gehalt an Reinigungskörpern lässt sich die Fließfähigkeit des Hautreinigungsmittels einstellen. Mit geringen Gehalten an Verdickungsmitteln und Reinigungskörpern werden Fließpasten erhalten. Mit relativ hohen Gehalten werden feste Reinigungspasten erhalten, wie sie beispielsweise zur Handreinigung häufig eingesetzt werden.

Überraschenderweise ist das erfindungsgemäße Produkt sehr hautfreundlich, da das eingesetzte Rizinusöl zusätzlich Eigenschaften als Rückfetter und Emulgator besitzt.

Die Reinigungswirkung der Reinigungskörper kann durch die Korngröße und das Herstellverfahren beeinflusst werden. Grundsätzlich geben größere Reinigungskörper eine stärkere Wirkung.

Hergestellt wird der erfindungsgemäß Reinigungskörper bevorzugt durch ein Verfahren, bei dem das hydrierte Rizinusöl, gegebenenfalls unter Zusatz weiterer Stoffe, insbesondere weiterer Wachse, geschmolzen wird und anschließend die Schmelze vertropft oder versprüht wird.

Beim Versprühen wird die geschmolzene Wachsmischung mit hohem Druck über eine Düsen versprüht. Die Kornverteilungskurve weist eine relativ breite Basis auf. Ferner sind die Tropfen durch Zusammenschmelzen ungleichmäßiger geformt.

Vertropfte Produkte weisen eine wesentlich engere Kornverteilungskurve auf. Die Produkte sind gleichmäßig rund und neigen wegen des weiteren Tropfenkegels nicht so stark zum Verkleben. Vertropfungseinrichtungen verwenden an Stelle der Sprühdüsen eine rotierende Scheibe. Auf dieser befinden sich definierte Bohrungen, die den Durchmesser der Teilchen bestimmen.

Die unregelmäßigen Körper haben bessere mechanische Reinigungseigenschaften, jedoch dadurch bedingt eine schlechtere Hautverträglichkeit.

Grundsätzlich ist es auch möglich, die erfindungsgemäßen Reinigungskörper auf Basis von hydriertem Rizinusöl mit Abrasivstoffen, beispielsweise mit Polyurethanreibkörpern zu mischenund in Hautreinigungsmitteln einzusetzen.

Gegenstand der Erfindung ist auch ein Reinigungskörper für kosmetische Präparate mit einer mittleren Korngröße von 100 bis 1.000 µm, die hydriertes Rizinusöl enthalten sowie ein Verfahren zur Herstellung der Reinigungskörper umfassend die Schritte des Schmelzens von hydriertem Rizinusöl, gegebenenfalls zusammen mit weiteren Substanzen, gefolgt von einem Vertropfen oder Versprühen der erhaltenen Schmelze.

Gegenstand ist weiterhin die Verwendung von Reinigungskörpern mit einer mittleren Korngröße von 100 bis 1.000 µm, die hydriertes Rizinusöl enthalten, zur Herstellung von Kosmetika, insbesondere Hautreinigungsmitteln.

Die Wachse können grundsätzlich auch kosmetische Wirkstoffe enthalten. Durch eine Kombination verschiedener Wachse lassen sich die Eigenschaften der Reinigungskörper gut einstellen.

| Wachs | Tropfpunkt [°C] | Penetration [0,1 mm] |
|---|---|---|
| | DIN 51801 | DIN 51579 |
| Carnaubawachs | 81 - 86 | < 1 |
| Candelillawachs | 68 - 73 | < 2 |
| Paraffinwachs (hart) | 105 - 120 | 1 - 4 |
| Paraffinwachs | 56 - 60 | 160 - 210 |
| Polyethylenwachs | 105 - 115 | 2 - 5 |
| Oxidiertes Polyethylenwachs | 101 - 109 | < 6 |
| Hydriertes Rizinus öl | 84 - 88 | 3 - 6 |

Erfindungsgemäße Reinigungskörper lassen sich auch erhalten, wenn statt des hydrierten oder teilweise hydrierten Rizinusöls ein Wachs eingesetzt wird, das ähnliche Eigenschaften hat, wie hydriertes Rizinusöl, insbesondere im Bereich der Härte und des Schmelzbereichs.

Bevorzugt ist die Abweichung bei Tropfpunkt und Penetration anderer Wachse nicht größer als 20% von den erfindungsgemäß einsetzbaren hydrierten Rizinusöl.

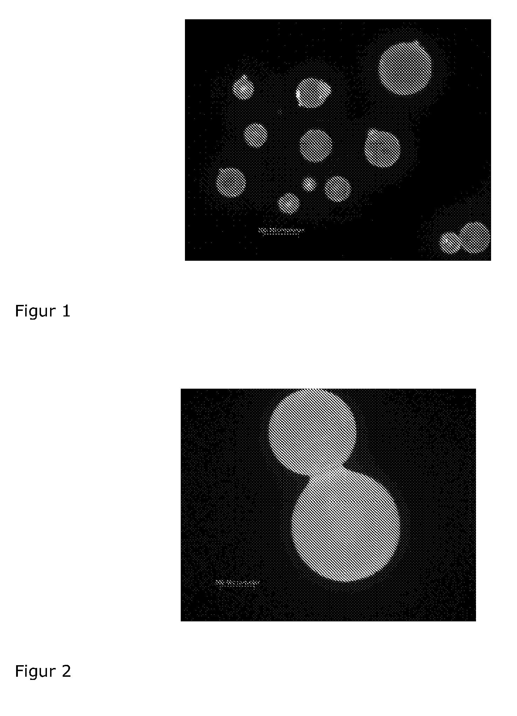

Figur 1 zeigt Reinigungskörper, wie sie aus dem Sprühverfahren erhalten werden.

Figur 2 zeigt Reinigungskörper, wie sie aus Vertropfungsverfahren erhalten werden.

Die Erfindung wird durch die nachfolgenden Beispiele näher erläutert.

### Beispiel 1

Ein Hautreinigungsmittel mit folgender Zusammensetzung:

| INCI EU | Anteil in % |
|---|---|
| Sodium C12-18 Alkylsulfate | 15,000000 |
| Aqua | 15,000000 |
| Paraffinum Liquidum | 18,000000 |
| Aqua | 16,500000 |
| Sodium C12-15 Pareth-Sulfate | 8,750000 |
| Aqua | 3,750000 |
| Hydrogenated Castor Oil | 10,000000 |
| Aqua | 3,000000 |
| Sodium Xylenesulfonate | 2,000000 |
| Glycerin | 3,00000 |
| Oleic Acid | 1,500000 |
| Sodium Chloride | 1,200000 |
| Coco-Glucoside | 0,330000 |
| Clyceryl Oleate | 0,330000 |
| Aqua | 0,340000 |
| Phenoxyethanol | 0,560000 |
| Butylparaben | 0,060000 |
| Ethylparaben | 0,060000 |
| Methylparaben | 0,060000 |
| Propylparaben | 0,060000 |
| Titanium Dioxide | 0,200000 |
| Parfüm | 0,200000 |
| Citric Acid | 0,100000 |

wurde hergestellt.

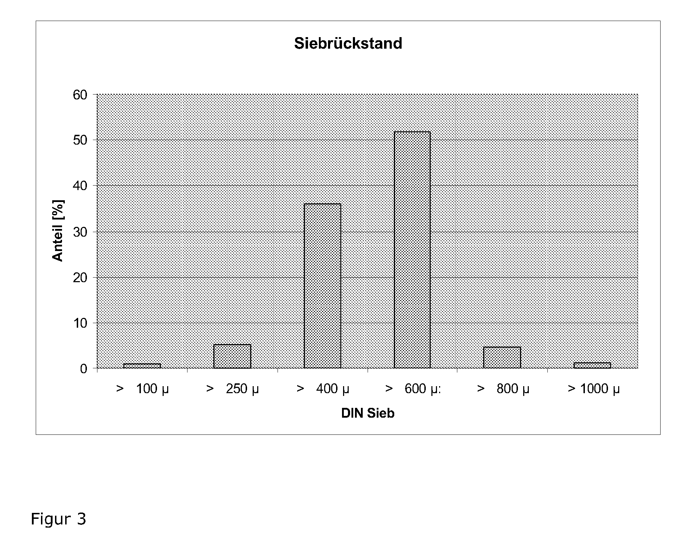

Das eingesetzte hydrierte Rizinusöl hatte eine Korngrößenverteilung gemäß Figur 3.

Es ergab sich eine relativ feste Hautreinigungspaste, die zur Entfernung starker Verschmutzung, wie Russ, Fette, Schmieröle geeignet ist. Es zeigte sich, dass die Reinigungskörper über einen längeren Zeitraum, auch unter erhöhten Temperaturbedingungen, wie sie beispielsweise bei Transport oder Lagerung im Sommer auftreten, stabil bleiben.

### Beispiel 2

| INCI EU | Anteli in % |
|---|---|
| AQUA | 60,5065 |
| SODIUM C12-15 PARETH SULFATE | 8,00 |
| HYDROGENATED CASTOR OIL (wie Beispiel 1) | 18,00 |
| SODIUM C12-18 ALKYL SULFATE | 5,00 |
| POTASSIUM COCOYL HYDROLYZED COLLAGEN | 1,00 |
| GLYCERYL RICINOLEATE | 2,00 |
| PEG-7 GLYCERYL COCOATE | 2,00 |
| BENTONITE | 2,50 |
| TITANIUM DIOXIDE | 0,60 |
| CITRIC ACID | 0,25 |
| PARFUM | 0,10 |
| 2-BROMO-2-NITROPROPANE-1,3-DIOL | 0,036 |
| CI 13015 | 0,002500 |
| CI 15510 | 0,002500 |
| CI 61570 | 0,002500 |

Das Beispiel 2 ist eine spenderfähige Fließpaste und kommt für den Bereich gröbster Verschmutzungen in Betracht. Die wirkungsvolle Tensidkombination wird durch die Verwendung von Eiweissfettsäurekondensate insgesamt wesentlich hautverträglicher. Die Reinigungswirkung wird durch das Reibemittel, das gerade für Schmierfette und -öle eine hohe Schmutzbindung aufweist, unterstützt. Rückfettende Substanzen mindern das Risiko von Hautirritationen.

### Beispiel 3

| INCI EU | Anteil in % |
|---|---|
| AQUA | 71,166 |
| SODIUM LAURETH SULFATE | 14,28 |
| Wachsmischung: | 5 |
| 85% Hydrogenated Castor oil | |
| 12% Paraffine | |
| 3% Carnauba wax | |
| COCAMIDOPROPYLBETAINE | 4,45 |
| PEG-7 GLYCERYL COCOATE | 2,00 |
| BENTONITE | 1,80 |
| CITRIC ACID | 0,55 |
| TITANIUM DIOXIDE | 0,50 |
| PARFUM | 0,10 |
| 2-BROMO-2-NITROPROPANE-1,3-DIOL | 0,054 |
| XANTHAN GUM | 0,10 |

Die Mischung hat einen Tropfpunkt von 78°C und eine Penetration von 214 bei 0,1 mm.

Das Beispiel 3 handelt es sich ebenfalls um eine Fließpaste, die bei einem mittleren Verschmutzungsgrad der Haut verwendet wird. Die Tensidkombination ist in ihrer Reinigungskraft gegenüber Beispiel 2 deutlich abgestuft. PEG-7 GLYCERYL COCOATE hat neben seinen exzellente Emulgier- und Schaumeigenschaften auch noch eine rückfettende Wirkung. Der pH-Wert ist dem der Haut angepasst.

### Beispiel 4

| INCI EU | Anteil in % |
|---|---|
| AQUA | 27,002 |
| HYDROGENATED CASTOR OIL (wie Beispiel 1) | 25,00 |
| SODIUM C12-18 ALKYL SULFATE | 23,73 |
| PARAFFINUM LIQUIDUM | 18,00 |
| POTASSIUM COCOYL HYDROLYZED COLLAGEN | 2,00 |
| CITRIC ACID | 2,00 |
| ISODECETH-7 | 1,00 |
| OLEIC ACID | 1,00 |
| PARFUM | 0,20 |
| 2-BROMO-2-NITROPROPANE-1,3-DIOL | 0,018 |
| TITANIUM DIOXIDE | 0,050 |

Das Beispiel 4 stellt eine feste bis pastenartige Handreinigungscreme dar. Sie ist für die Entfernung gröbster Verschmutzungen, wie Ruß, Schmierfette, Bitumen etc konzipiert. Die hohe Reinigungskraft begründet sich zum einen durch den Gehalt an Paraffinöl (DAB), zum anderen durch die wirkungsvolle Tensidkombination. Trotz des relativ hohen Anteils an Paraffin bleibt das Reinigungsmittel über Monate hin stabil.

### Beispiel 5

| INCI EU | Anteil in % |
|---|---|
| DIMETHYL GLUTARATE | 27,0 |
| DIMETHYL SUCCINATE | 8,3 |
| DIMETHYL ADIPATE | 6,2 |
| AQUA | 11,0 |
| DISODIUM LAURETH SULFOSUCCINATE | 9,2 |
| AQUA | 13,2 |
| HYDROGENATED CASTOR OIL (wie Beispiel 1) | 11,5 |
| ETHYLHEXYL STEARATE | 4,6 |
| PARAFFINUM LIQUIDUM | 4,6 |
| STEARALKONIUM BENTONITE | 3,0 |
| BENTONITE | 1,0 |
| PARFUM | 0,2 |
| TITANIUM DIOXIDE | 0,2 |
| | 100 |

Die Beispielrezeptur 5 gibt ein pastöses Spezialhandreinigungsmittel für die Entfernung stark haftender Verschmutzungen wie Bitumen, Lacke oder Klebstoffe wieder. Dieser starke Reinigungseffekt wird durch die Verwendung eines dibasischen Esters erreicht. Als hautverträgliches Tensid kommt ein Succinat zum Einsatz. Trotz dieses Inhaltstoffes bleibt das Reinigungsmittel stabil.

## Patentansprüche

1. Hautreinigungsmittel enthaltend
- 2 bis 25 Gew.-% Reinigungskörper mit einer mittleren Korngröße von 100 bis 1.000 µm enthaltend hydriertes Rizinusöl,
- 2 bis 30 Gew.-% Tenside,
- 0,1 bis 10 Gew.-% Verdickungsmittel,
- Wasser, sowie gegebenenfalls weiteren Hilfsstoffe.

2. Hautreinigungsmittel nach Anspruch 1, **dadurch gekennzeichnet, dass** das Rizinusöl vollständig oder partiell hydriert ist.

3. Hautreinigungsmittel nach einem der Ansprüche 1 oder 2, **dadurch gekennzeichnet, dass** die Reinigungskörper zusätzlich Paraffinwachse, Carnaubawachse, Candelilawachse, Polyethylenwachse, oxidierte Polyethylenwachse oder Mischungen davon mit einem Schmelzpunkt oberhalb von 40°C enthalten.

4. Hautreinigungsmittel nach Anspruch 1, **dadurch gekennzeichnet, dass** das Verdickungsmittel aus Bentoniten, Xanthanen, Acrylaten, Alginate, Celluloseether, Carrageen und Mischungen davon ausgewählt wird.

5. Hautreinigungsmittel nach mindestens einem der Ansprüche 1 bis 4, **dadurch gekennzeichnet, dass** zusätzlich eine oder mehrere der folgenden Substanzen enthalten sind
- Rückfetter,
- Farbstoffe,
- Parfümstoffe,
- Titandioxid,
- Puffersubstanzen,
- Konservierungsmittel,
- flüssige Parafine,
- Glycerin,
- Antioxidationsmitteln,
- Abrasiva
enthaltend sind.

6. Reinigungskörper für kosmetische Präparate, insbesondere Hautreinigungsmittel, mit einer mittleren Korngröße von 100 bis 1.000 µm, die hydriertes Rizinusöl enthalten.

7. Verfahren zur Herstellung der Reinigungskörper nach Anspruch 6 umfassend die Schritte
- des Schmelzens von hydriertem Rizinusöl gegebenenfalls zusammen mit weiteren Wachsen
- Vertropfen oder Versprühen der Schmelze.

8. Verwendung von Reinigungskörpern mit einer mittleren Korngröße von 100 bis 1.000 µm, die hydriertes Rizinusöl enthalten, zur Herstellung von Kosmetika, insbesondere Hautreinigungsmitteln.
